# EUROPEAN PATENT APPLICATION

(11) **EP 0 884 061 A2**
(43) Date of publication of application: **16.12.1998**
(21) Application number: 98303832.4
(22) Date of filing: 15.05.1998
(51) Int. Cl.: A61M 16/04

(54) **Cuffed tubes**

(30) Priority: 13.06.1997 GB 9712326
(71) Applicant: Smiths Industries Public Limited Company, London, NW11 8DS (GB)
(72) Inventor: Pagan, Eric, Hythe, Kent CT21 6DA (GB)
(74) Representative: Flint, Jonathan McNeill

(57) **Abstract**

The inflatable cuff (2) on an endotracheal tube has several annular channels (20, 21) around its outside along a central region of the cuff. The channels (20, 21) have a keyway shape and are produced by applying reinforcing bands (40, 41) to the surface of the cuff (2) so that, when inflated, expansion is restricted in the region of the channels.

## Description

This invention relates to cuffed tubes of the kind comprising a tubular shaft and an expansible cuff surrounding the shaft, the external surface of the cuff having at least one annular channel.

Various medico-surgical tubes, such as tracheal tubes, have a cuff encompassing the tube, which can be inflated to seal with the body cavity in which the tube is inserted. In this way, flow is confined along the tube. The cuffs take various different forms. Usually, they have a cylindrical or rounded shape, or they may have a pear shape, such as in the tube sold under the Profile trade mark by SIMS Portex Limited. It has been proposed previously that a cuff be moulded with a series of annular corrugations, as described in US3810474, to form a labyrinth seal between the trachea and the tube. These cuffs are moulded with the desired shape, but it is difficult to retain the shape, when the cuff is inflated and in contact with the tissue of the trachea, because of variations and changes in diameter of the trachea.

It is an object of the present invention to provide an improved cuffed tube.

According to the present invention there is provided a cuffed medico-surgical tube of the above-specified kind, characterised in that the or each channel has a reinforcement band around the cuff that helps define the shape of the channel when expanded.

The cuff may have a plurality of annular channels. The cuff may have a central region between two end regions attached with the shaft, the or each channel being located in the central region and the central region tapering along its length to a smaller diameter away from the patient end of the tube. The floor of the or each channel may be wider than the mouth when the cuff is in an expanded state. The or each reinforcement band may be produced by applying a substance in liquid form to a surface of the cuff. Alternatively, the or each reinforcement band may be produced by a strip of material applied to a surface of the cuff. The or each reinforcement band preferably has weakened regions around its circumference to facilitate controlled collapse of the cuff. The or each reinforcement band may be on an external surface of the cuff. Alternatively, the or each reinforcement band may be applied to an external surface of the cuff, the cuff then being inverted so that the or each reinforcement band is on an internal surface of the cuff. The cuff preferably has a central region between two end regions attached with the shaft, the end regions being reinforced to restrict axial displacement of the cuff.

A cuffed endotracheal tube according to the present invention, will now be described, by way of example, with reference to the accompanying drawings, in which:
- Figure 1: is a side elevation view of the tube;
- Figure 2: is a cross sectional side elevation view of the patient end of the tube to a larger scale;
- Figure 3: is a perspective view of the patient end of the tube;
- Figure 4: is a perspective view of a reinforcing band of the cuff; and
- Figures 5 and 6: are a cross-sectional side elevation views of two alternative tubes.

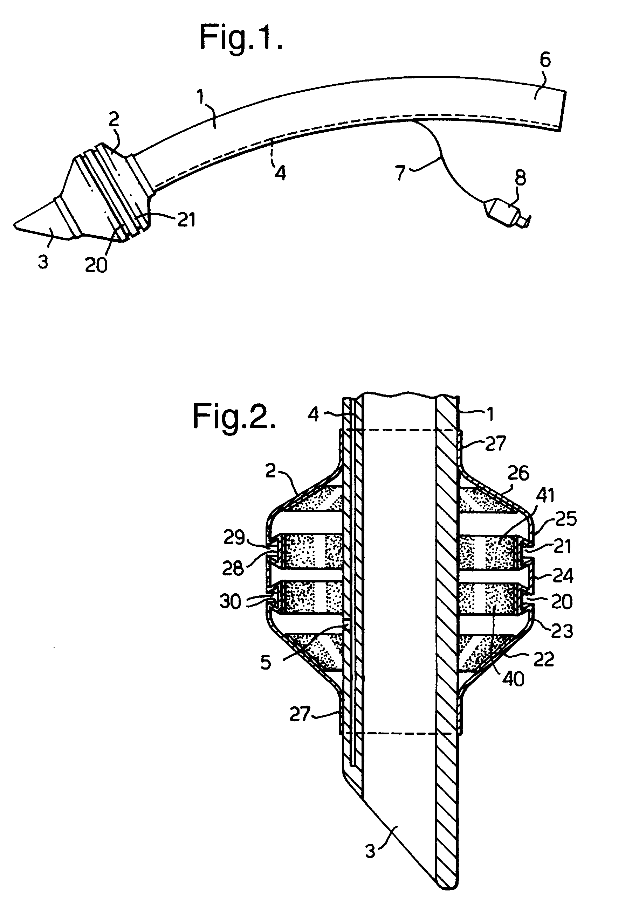

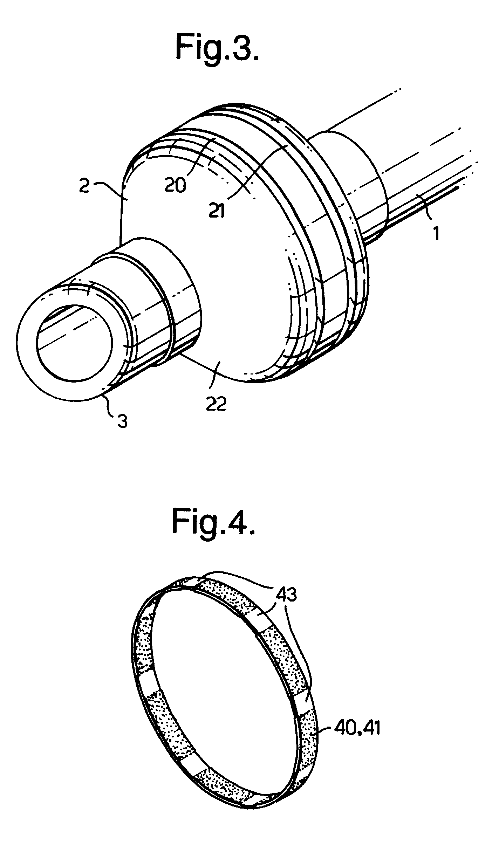

With reference first to Figures 1 to 4, the tube has a curved tubular shaft 1 of PVC or similar plastics material with a cuff 2 surrounding the shaft close to its patient end 3. The shaft has an inflation lumen 4 extruded within its wall, which communicates with the interior of the cuff 2 via an opening 5 in the outer surface of the wall of the shaft. Towards the machine end 6 of the tube, the inflation lumen 4 connects with one end of a small diameter inflation line 7, the other end of which is terminated by a combined inflation indicator and connector 8. As so far described, the tube is conventional.

The cuff 2 of the tube, however, differs from conventional tubes in that it has two annular channels 20 and 21 formed around its exterior surface and spaced from one another along the length of the cuff. These channels divide the cuff 2 into the following regions: a patient-end tapering region 22, a patient-end contact region 23, the first, patient-end channel 20, an intermediate contact region 24, the second, machine-end channel 21, a machine-end contact region 25 and a machine-end tapering region 26. The cuff 2 also has a collar 27 at each end sealed to the outside of the shaft. The inflated diameter of the cuff 2 is typically about 3 5mm with the central region provided by the three contact regions 23, 24 and 25 reducing in diameter from the patient end, so that the cuff tapers externally along its central region.

The channels 20 and 21 have a keyway shape section, that is, the floor 28 of the channel is wider than the mouth 29, with the two sides 30 of the channel being inclined inwardly towards the extemal surface. The channels could have other shapes. For example, they could have a square or V-shape. Alternatively, they could have a tapered section, with a flat floor and outwardly inclined sides.

The channels 20 and 21 are produced by forming two reinforced bands 40 and 41 around the cuff so as to prevent these reinforced regions expanding outwardly to the same extent as the adjacent regions on either side. The reinforcement bands 40 and 41 may be produced in various different ways but are preferably produced by applying a stiffer variant of the substrate or a different substance, such as polyurethane, onto the surface of the cuff. This may be done by, for example, printing, painting or spraying the substance in liquid form onto the outside of the cuff in the desired regions. Alternatively, a strip of reinforcing material could be bonded to the cuff such as by laminating or fabricating, or during the moulding process of the cuff. The cuff could be inverted after application of the reinforcement bands so that these are located on the inside, with the outside surface of the cuff being maintained smooth. The keyway shape of the channels is produced by appropriately selecting the width of the reinforcement bands 40 and 41, the length of the cuff 2 and the mounting locations of the collars 27. The reinforcement bands 40 and 41 form the floor of the channels 20 and 21 so that these are maintained relatively flat; the tapering end regions 22 and 26 of the cuff 2 are also reinforced so that these are stiffer than the unreinforced regions 23, 24 and 25 and they restrict axial movement of the cuff on inflation. In this way, the unreinforced regions 23, 24 and 25 expand to overlap the floor of the channels 20 and 21, when the cuff 2 is inflated, giving the keyway shape.

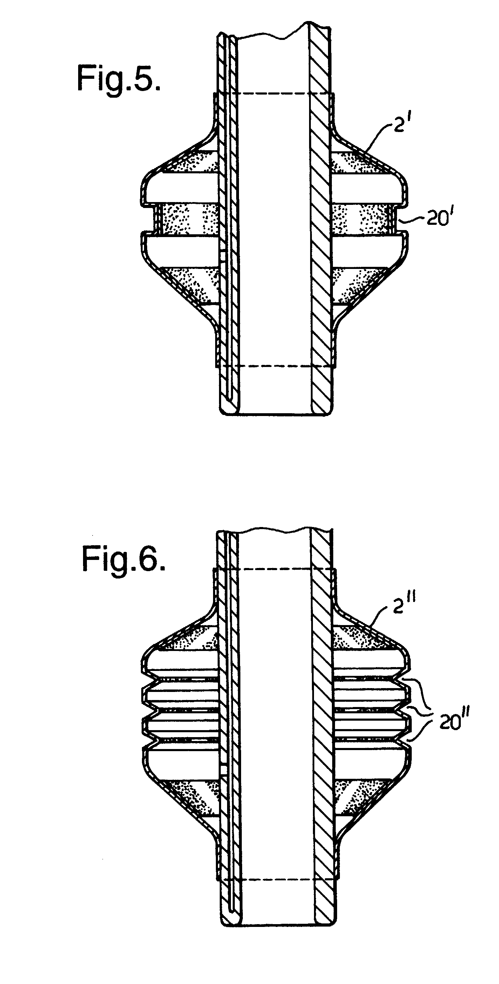

As shown in Figure 4, each reinforcing band 40 and 41 has several weak regions 43 around its circumference, so as to facilitate controlled collapse of the cuff 2, when it is deflated.

The cuff 2, when inflated, forms an effective seal with the surface of the trachea because it is less prone to creases than conventional uncorrugated cuffs and retains the shape of its annular corrugations effectively while in use. This also helps form an improved labyrinth seal with the trachea.

Various other forms of cuff are possible. For example, different numbers of annular channels could be provided. Figure 5 shows a cuff 2' with only one channel 20', this having a square section. Figure 6 shows a cuff 2" with three channels 20", which are of V-shape in section. The cuff need not be inflated by introducing air but could, for example, contain a resilient material so that it has a naturally expanded state and is reduced in size for insertion and removal by applying a negative, vacuum pressure.

The invention is not restricted to tracheal tubes but could be used in other cuffed medico-surgical tubes.

## Claims

1. A cuffed medico-surgical tube comprising a tubular shaft (1) and an expansible cuff (2, 2', 2") surrounding the shaft, the external surface of the cuff having at least one annular channel (20, 21, 20', 20"), characterised in that the or each channel (20, 21, 20', 20'') has a reinforcement band (40, 41) around the cuff that helps define the shape of the channel when expanded.

2. A tube according to Claim 1, characterised in that the cuff (2, 2") has a plurality of annular channels (20, 21, 20").

3. A tube according to Claim 1 or 2, in which the cuff (2, 2") has a central region (23, 24, 25) between two end regions (22 and 26) attached with the shaft (1), the or each channel (20, 21, 20', 20") being located in the central region (23, 24, 25), characterised in that the central region (23, 24, 25) tapers along its length to a smaller diameter away from the patient end of the tube.

4. A tube according to any one of the preceding claims, characterised in that the floor (28) of the or each channel (20, 21) is wider than the mouth (29) when the cuff (2) is in an expanded state.

5. A tube according to any one of the preceding claims, characterised in that the or each reinforcement band (40, 41) is produced by applying a substance in liquid form to a surface of the cuff (2, 2', 2").

6. A tube according to any one of Claims 1 to 4, characterised in that the or each reinforcement band (40, 41) is produced by a strip of material applied to a surface of the cuff (2, 2', 2").

7. A tube according to any one of the preceding claims, characterised in that the or each reinforcement band (40, 41) has weakened regions (43) around its circumference to facilitate controlled collapse of the cuff (2, 2', 2'').

8. A tube according to any one of the preceding claims, characterised in that the or each reinforcement band (40, 41) is on an outer surface of the cuff.

9. A tube according to any one of the Claims 1 to 7, characterised in that the or each reinforcement band (40, 41) is applied to an outer surface of the cuff (2, 2', 2'') and the cuff is then inverted so that the or each reinforcement band is on the inner surface of the cuff.

10. A tube according to any one of the preceding claims, characterised in that the cuff (2, 2', 2") has a central region (23, 24, 25) between two end regions (22 and 26) attached with the shaft, and that the end regions are reinforced to restrict axial displacement of the cuff.
